(19) Europäisches Patentamt
European Patent Office
Office européen des brevets

(11) EP 0 891 975 A1

(12) **EUROPEAN PATENT APPLICATION**
published in accordance with Art. 158(3) EPC

(43) Date of publication:
**20.01.1999 Bulletin 1999/03**

(21) Application number: **97914601.6**

(22) Date of filing: **04.04.1997**

(51) Int. Cl.$^6$: **C07D 401/12**, C07D 401/14, C07D 405/14, C07D 417/14, A01N 43/56, A01N 43/78

(86) International application number:
**PCT/JP97/01162**

(87) International publication number:
**WO 97/37990 (16.10.1997 Gazette 1997/44)**

(84) Designated Contracting States:
**DE DK ES FR GB IE IT NL PT**

(30) Priority: **05.04.1996 JP 83587/96**
**09.08.1996 JP 211377/96**

(71) Applicant:
**MITSUBISHI CHEMICAL CORPORATION**
**Chiyoda-ku, Tokyo 100-0005 (JP)**

(72) Inventors:
- **KYOMURA, Nobuo**
  **Aoba-ku, Yokohama-shi, Kanagawa 227 (JP)**
- **IKEDA, Yoshiya**
  **Aoba-ku, Yokohama-shi, Kanagawa 227 (JP)**
- **OKUI, Shuko**
  **Aoba-ku, Yokohama-shi, Kanagawa 227 (JP)**
- **TOMITA, Hirofumi**
  **Aoba-ku, Yokohama-shi, Kanagawa 227 (JP)**
- **HIGASHINO, Yoshiaki**
  **Aoba-ku, Yokohama-shi, Kanagawa 227 (JP)**
- **KOIKE, Sanae**
  **Aoba-ku, Yokohama-shi, Kanagawa 227 (JP)**

(74) Representative:
**VOSSIUS & PARTNER**
**Siebertstrasse 4**
**81675 München (DE)**

(54) **PYRAZOLES AND AGRICULTURAL CHEMICALS CONTAINING THEM AS ACTIVE INGREDIENTS**

(57) A pyrazole compound represented by formula (I):

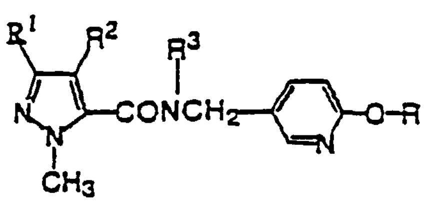

(I)

wherein $R^1$ represents a hydrogen atom, a $C_{1-4}$ alkyl group, a $C_{1-4}$ haloalkyl group, a $C_{1-4}$ alkoxy group or a phenyl group; $R^2$ represents a hydrogen atom, a halogen atom, a hydroxyl group, a $C_{1-4}$ alkyl group, a $C_{1-4}$ alkoxy group, a $C_{1-4}$ haloalkoxy group, a $C_{2-8}$ alkoxyalkoxy group or a benzyloxy group; or $R_1$ and $R_2$ are taken together to form a group:

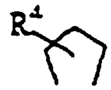

EP 0 891 975 A1

wherein $R^4$ represents a hydrogen atom or a $C_{1-4}$ alkyl group;

$R^3$ represents a hydrogen atom, a $C_{1-4}$ alkyl group, a $C_{2-5}$ alkenyl group, a $C_{5-7}$ cycloalkenyl group, a $C_{2-5}$ alkoxyalkyl group, a $C_{8-12}$ benzyloxyalkyl group, a formyl group, a $C_{2-5}$ alkanoyl group, a benzoyl group, a $C_{2-5}$ alkoxycarbonyl group, a benzyloxycarbonyl group, a $C_{1-4}$ alkylsulfonyl group or a $C_{6-10}$ arylsulfonyl group; and R represents a group represented by formulae:

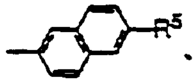 , 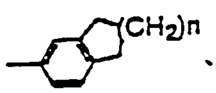 , 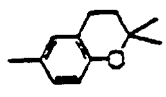 , 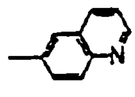 , 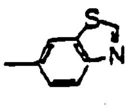 or 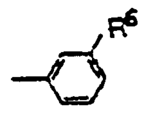

wherein $R^5$ represents a hydrogen atom, a halogen atom or a $C_{1-4}$ alkoxy group; $R^6$ represents a halogen atom, a $C_{1-4}$ alkyl group, a $C_{1-4}$ haloalkyl group, a $C_{1-4}$ alkoxy group or a $C_{1-4}$ haloalkoxy group; and n represents 1 or 2, and a pesticide, such as a fungicide, a bacteriocide, an insecticide or an acaricide, comprising the same as an active ingredient. The pyrazole compound exhibits higher pesticidal activities than conventional pesticidal compounds and are of high safety.

## Description

Technical Field:

This invention relates to a novel pyrazole compound and a pesticide (e.g., fungicides, bacteriocides, insecticides and acaricides) containing the same.

Background Art:

In agriculture and horticulture, a wide variety of pesticides such as fungicides, bacteriocides, insecticides and acaricides have been developed and put to practical use for the purpose of controlling pests.

However, currently spread agricultural and horticultural fungicides, bacteriocides, insecticides and acaricides are not necessarily satisfactory in pesticidal effect, pesticidal spectrum, residual activity, and the like. Further they may be seen as inadequate to meet the demand for reducing the times of application or the amount to be applied.

On the other hand, emergence of strains of pathogenic microorganisms or insect pests which have acquired resistance to the conventional pesticides has now become a serious problem. For example, pathogenic microorganisms or insect pests having developed resistance to various types of pesticides, such as triazole compounds, imidazole compounds, pyrimidine compounds, benzimidazole compounds, dicarboxyimide compounds, phenylamide compounds and organophosphorus compounds, have come out in various sites of cultivation of vegetables, fruits, flowers, teas, grains, rices, etc. Control of various plant diseases or damages causes by these pathogens or insect pests is becoming increasingly difficult year by year.

While there are some pesticides to which pathogens or insect pests have not yet acquired resistance, such as dithiocarbamate compounds and phthalimide compounds, they are not preferred from the viewpoint of environmental pollution because, in general, they should be applied in large amounts or frequently.

Accordingly, it has been keenly demanded to develop a novel fungicide, bacteriocide or insecticide which exhibits sufficient controlling effects at a low amount on various pathogenic fungi or bacteria and insect pests having acquired resistance to the conventional agricultural or horticultural fungicides, bacteriocides and insecticides with minimal influences on the environment. As for acaricides, too, it has been expected to develop an acaricide which exhibits an excellent controlling effect on mites having resistance against conventional acaricides while securing safety.

JP-A-7-173139 (the term "JP-A" as used herein means an "unexamined published Japanese patent application") discloses N-(substituted pyridylmethyl)pyrazolecarboxamide compounds, teaching that the compounds have fungicidal, bactericidal, insecticidal and acaricidal activities. However, the substituents on the pyridine ring disclosed in the publication are limited to acyclic groups.

N-(Phenoxypyridylmethyl)pyrazolecarboxamide compounds are proposed in JP-A-2-62876 as compounds having fungicidal, bactericidal, insecticidal and acaricidal activities. However, almost all the compounds disclosed are those having a substituent on the 4-position of the phenoxy moiety, and there is presented only one 3,4-disubstituted compound as a compound having a substituent on the 3-position of the phenoxy moiety.

An object of the present invention is to provide a chemical substance which exhibits high controlling effects on various pathogens and insect pests showing resistance to the conventional agricultural or horticultural pesticides and is useful as an active ingredient of pesticides such as fungicides, bacteriocides, insecticides and acaricides. Another object of the present invention is to provide highly safe pesticides which have the above-mentioned characteristics and yet are less involved in the problems of residual toxicity and environmental pollution.

In order to achieve the above objects, the inventors of the present invention have carried out extensive studies and, as a result, found that a pyrazole compound represented by the formula shown below which possesses (1) a phenoxy group having a specific condensed ring or (2) a phenoxy group having a specific substituent on the meta-position thereof bears all the above-described characteristics. The present invention has been completed based on this finding.

Disclosure of the Invention:

The gist of the present invention consists in a pyrazole compound represented by formula (I) and a pesticide comprising the compound as an active ingredient:

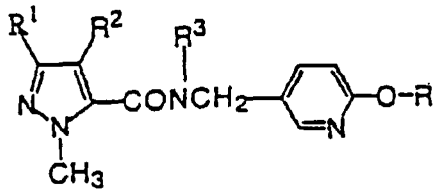

(I)

wherein $R^1$ represents a hydrogen atom, a $C_{1-4}$ alkyl group, a $C_{1-4}$ haloalkyl group, a $C_{1-4}$ alkoxy group or a phenyl group; $R^2$ represents a hydrogen atom, a halogen atom, a hydroxyl group, a $C_{1-4}$ alkyl group, a $C_{1-4}$ alkoxy group, a $C_{1-4}$ haloalkoxy group, a $C_{2-8}$ alkoxyalkoxy group or a benzyloxy group; or $R_1$ and $R_2$ are taken together to form a group:

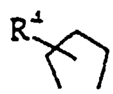

wherein $R^4$ represents a hydrogen atom or a $C_{1-4}$ alkyl group;

$R^3$ represents a hydrogen atom, a $C_{1-4}$ alkyl group, a $C_{2-5}$ alkenyl group, a $C_{5-7}$ cycloalkenyl group, a $C_{2-5}$ alkoxyalkyl group, a $C_{8-12}$ benzyloxyalkyl group, a formyl group, a $C_{2-5}$ alkanoyl group, a benzoyl group, a $C_{2-5}$ alkoxycarbonyl group, a benzyloxycarbonyl group, a $C_{1-4}$ alkylsulfonyl group or a $C_{6-10}$ arylsulfonyl group; and R represents a group represented by formulae:

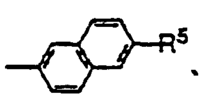, 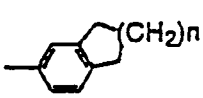, 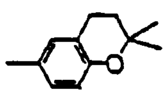, 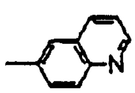, 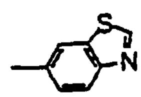 or 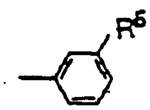

wherein $R^5$ represents a hydrogen atom, a halogen atom or a $C_{1-4}$ alkoxy group; $R^6$ represents a halogen atom, a $C_{1-4}$ alkyl group, a $C_{1-4}$ haloalkyl group, a $C_{1-4}$ alkoxy group or a $C_{1-4}$ haloalkoxy group; and n represents 1 or 2.

Hereinafter, the present invention is explained in detail.

The pyrazole compounds according to the present invention are compounds represented by formula (I), wherein:

$R^1$ represents a hydrogen atom; a $C_{1-4}$ straight-chain or branched alkyl group, such as methyl, ethyl, n-propyl, isopropyl, n-butyl, isobutyl, sec-butyl or t-butyl; a $C_{1-4}$ straight-chain or branched haloalkyl group, such as difluoromethyl, trifluoromethyl, 2-fluoroethyl, 2-chloroethyl, 2,2,2-trifluoroethyl, 2,2,2-trichloroethyl, 3-chloropropyl, 3-bromopropyl, 3,3,3-trifluoropropyl, 3,3,3-trichloropropyl, 2,2,3,3-tetrafluoropropyl, 2,2,3,3,3-pentafluoropropyl group, 2,2-dichloro-3,3,3-trifluoropropyl, 1,3-difluoro-2-propyl, 1,1,1,3,3,3-hexafluoro-2-propyl, 4-chlorobutyl, 4,4,4-trifluorobutyl or 3,3,4,4,4-pentafluorobutyl; a $C_{1-4}$ straight-chain or branched alkoxy group, such as methoxy, ethoxy, n-propoxy, isopropoxy, n-butoxy, isobutoxy, sec-butoxy or t-butoxy; or a phenyl group.

$R^2$ represents a hydrogen atom; a halogen atom, such as fluorine, chlorine or iodine; a hydroxyl group; a $C_{1-4}$ straight-chain or branched alkyl group, such as methyl, ethyl, n-propyl, isopropyl, n-butyl, isobutyl, sec-butyl or t-butyl; a $C_{1-4}$ straight-chain or branched alkoxy group, such as methoxy, ethoxy, n-propoxy, isopropoxy, n-butoxy, isobutoxy, sec-butoxy or t-butoxy; a $C_{1-4}$ straight-chain or branched haloalkoxy group, such as difluoromethoxy, trifluoromethoxy, 2-fluoroethoxy, 2-chloroethoxy, 2,2,2-trifluoroethoxy, 2,2,2-trichloroethoxy, 3-chloropropoxy, 3-bromopropoxy, 3,3,3-trifluoropropoxy, 2,2,3,3-tetrafluoropropoxy, 2,2,3,3,3-pentafluoropropoxy, 2,2-dichloro-3,3,3-trifluoropropoxy, 1,3-difluoro-2-propoxy, 1,1,1,3,3,3-hexafluoro-2-propoxy, 3,3,3-trichloropropoxy, 4-chlorobutoxy, 4,4,4-trifluorobutoxy or 3,3,4,4,4-pentafluorobutoxy; or a benzyloxy group.

$R^1$ and $R^2$ may be taken together to form a group represented by formula:

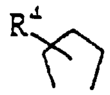

wherein $R^4$ represents a hydrogen atom or a $C_{1-4}$ straight-chain or branched alkyl group, such as methyl, ethyl, n-propyl, isopropyl, n-butyl, isobutyl, sec-butyl or t-butyl. Of the groups formed by $R^1$ and $R^2$ connected together, a group represented by formula:

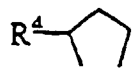

is preferred, in which $R^4$ is at the 6-position of the condensed pyrazole ring.

$R^3$ represents a hydrogen atom; a $C_{1-4}$ straight-chain or branched alkyl group, such as methyl, ethyl, n-propyl, isopropyl, n-butyl, isobutyl, sec-butyl or t-butyl; a $C_{2-5}$ straight-chain or branched alkenyl group, such as vinyl, propenyl, butenyl or pentenyl; a $C_{5-7}$ cycloalkenyl group, such as cyclopentenyl, cyclohexenyl or cycloheptenyl; a $C_{2-5}$ straight-chain or branched alkoxyalkyl group, such as methoxymethyl, ethoxymethyl, isopropoxymethyl, methoxyethyl, ethoxyethyl, methoxypropyl or ethoxypropyl; a $C_{8-12}$ benzyloxyalkyl group, such as benzyloxymethyl, benzyloxyethyl or benzyloxypropyl; a formyl group; a $C_{2-5}$ alkanoyl group, such as acetyl, propionyl, n-butyryl, n-valeryl, isovaleryl, 2-methylbutyryl or pivaloyl; a benzoyl group; a $C_{2-5}$ alkoxycarbonyl group, such as methoxycarbonyl, ethoxycarbonyl, n-propoxycarbonyl, isopropoxycarbonyl, n-butoxycarbonyl, isobutyoxycarbonyl, sec-butoxycarbonyl or t-butoxycarbonyl; a benzyloxycarbonyl group; a $C_{1-4}$ alkylsulfonyl group, such as methanesulfonyl or ethanesulfonyl; or a $C_{6-10}$ arylsulfonyl group, such as p-toluenesulfonyl. $R^3$ is preferably a hydrogen atom, a $C_{2-5}$ alkenyl group, a $C_{5-7}$ cycloalkenyl group, a $C_{2-5}$ alkoxyalkyl group, a benzyloxymethyl group, a formyl group, a $C_{2-5}$ alkanoyl group or a p-toluenesulfonyl group, with a hydrogen atom, a formyl group or an alkanoyl group being particularly preferred.

$R^5$ represents a hydrogen atom; a halogen atom, such as fluorine, chlorine or iodine; or a $C_{1-4}$ straight-chain or branched alkoxy group, such as methoxy, ethoxy, n-propoxy, isopropoxy, n-butoxy, isobutoxy, sec-butoxy or t-butoxy.

$R^6$ represents a halogen atom, such as fluorine, chlorine or iodine; a $C_{1-4}$ straight-chain or branched alkyl group, such as methyl, ethyl, n-propyl, isopropyl, n-butyl, isobutyl, sec-butyl or t-butyl; a $C_{1-4}$ straight-chain or branched haloalkyl group, such as difluoromethyl, trifluoromethyl, 2-fluoroethyl, 2-chloroethyl, 2,2,2-trifluoroethyl, 2,2,2-trichloroethyl, 3-chloropropyl, 3-bromopropyl, 3,3,3-trifluoropropyl, 2,2,3,3-tetrafluoropropyl, 2,2,3,3,3-pentafluoropropyl group, 2,2-dichloro-3,3,3-trifluoropropyl, 1,3-difluoro-2-propyl, 1,1,1,3,3,3-hexafluoro-2-propyl, 3,3,3-trichloropropyl, 4-chlorobutyl, 4,4,4-trifluorobutyl or 3,3,4,4,4-pentafluorobutyl; a $C_{1-4}$ straight-chain or branched alkoxy group, such as methoxy, ethoxy, n-propoxy, isopropoxy, n-butoxy, isobutoxy, sec-butoxy or t-butoxy; or a $C_{1-4}$ straight-chain or branched haloalkoxy group, such as difluoromethoxy, trifluoromethoxy, 2-fluoroethoxy, 2-chloroethoxy, 2,2,2-trifluoroethoxy, 2,2,2-trichloroethoxy, 3-chloropropoxy, 3-bromopropoxy, 3,3,3-trifluoropropoxy, 2,2,3,3-tetrafluoropropoxy, 2,2,3,3,3-pentafluoropropoxy, 2,2-dichloro-3,3,3-trifluoropropoxy, 1,3-difluoro-2-propoxy, 1,1,1,3,3,3-hexafluoro-2-propoxy, 3,3,3-trichloropropoxy, 4-chlorobutoxy, 4,4,4-trifluorobutoxy or 3,3,4,4,4-pentafluorobutoxy.

$R^6$ is preferably a halogen atom, a $C_{1-4}$ alkyl group, a $C_{1-4}$ haloalkyl group, a $C_{1-4}$ alkoxy group or a $C_{1-4}$ haloalkoxy group, with a halogen atom or a $C_{1-4}$ haloalkyl group being particularly preferred.

Where R is an meta-substituted phenoxy group, $R^1$ is preferably a $C_{1-4}$ alkyl group or a $C_{1-4}$ haloalkyl group, and $R^2$ is preferably a hydrogen atom, a halogen atom, a $C_{1-4}$ alkyl group or a $C_{1-4}$ alkoxy group.

Where R is a condensed phenoxy group, $R^1$ is preferably a hydrogen atom or a $C_{1-4}$ alkyl group, and $R^2$ is preferably a halogen atom, a $C_{1-4}$ alkyl group or a $C_{1-4}$ alkoxy group.

The compounds represented by formula (I) are prepared, for example, in accordance with the following reaction formula:

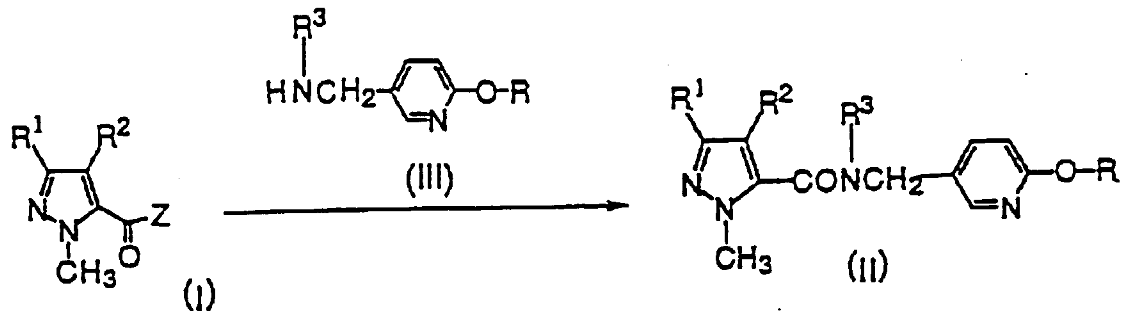

wherein $R^1$, $R^2$, $R^3$, and R are as defined above; and Z represents a chlorine atom, a bromine atom, a hydroxyl group or a lower alkoxy group, such as methoxy, ethoxy or propoxy.

The compound (III) is used in an amount of 0.5 to 2.0 mol, preferably 0.8 to 1.5 mol, per mole of the compound (II).

Where Z in formula (II) is a chlorine atom or a bromine atom, the above reaction is carried out at 0° to 30°C, preferably 0° to 5°C, for 1 to 3 hours in a solvent in the presence of a base. Suitable solvents include aromatic hydrocarbons such as benzene, toluene and xylene; ketones such as acetone, methyl ethyl ketone and methyl isobutyl ketone; halogenated hydrocarbons such as chloroform and methylene chloride; water; esters such as methyl acetate and ethyl acetate; and polar solvents such as tetrahydrofuran, acetonitrile, dioxane, N,N-dimethylformamide, N-methylpyrrolidone, and dimethyl sulfoxide. Preferred of them are aromatic hydrocarbons, esters, tetrahydrofuran, acetonitrile, and dioxane. The solvent is used in an amount 3 to 30 times, preferably 5 to 20 times, the weight of the compound (III). Suitable bases include commonly employed inorganic bases, such as alkali metal hydroxides (e.g., sodium hydroxide and potassium hydroxide), alkaline earth metal hydroxides (e.g., magnesium hydroxide and calcium hydroxide), alkali metal oxides, and alkali metal carbonates; and organic bases, such as triethylamine, pyridine, 1,4-diazabicyclo[2,2,2]octane, 1,5-diazabicyclo[4,3,0]non-5-ene, and 1,8-diazabicyclo[5,4,0]undec-7-ene. An alkali metal hydroxide, triethylamine or pyridine is preferred. The base is used in an amount of 0.5 to 2.0 mol, preferably 0.8 to 1.5 mol, per mole of the compound (II).

Where Z in formula (II) is a hydroxyl group, the reaction is conducted at 150° to 250°C, preferably 200° to 250°C, in a solvent under atmospheric pressure or under pressure. Suitable solvents include high-boiling solvents such as N,N-dimethylformamide, N-methylpyrrolidone, and dimethyl sulfoxide. Where Z in formula (II) is a lower alkoxy group, the reaction is performed at 150° to 250°C, preferably 200° to 250°C, with or without a solvent under atmospheric pressure or under pressure. Suitable solvents, if used, include high-boiling aprotic polar solvents such as N,N-dimethylformamide, N-methylpyrrolidone, and dimethyl sulfoxide.

The amount of the solvent, if used, is 3 to 30 times, preferably 5 to 20 times, the weight of the compound (III).

The compound (II) is prepared, for example, in accordance with the process described in *Bull. Soc. Chim. France*, 293 (1966) or *Annalen der Chemie, Justus Liebig's,* 536, 97 (1938). The compound (III) is prepared by, for example, the process described in *Journal fur Praktische Chemie*, 146, 95 (1936).

The compounds represented by formula (I) according to the present invention show high fungicidal effects on plant pathogenic fungi, such as blast fungi, rust fungi, and downy mildew fungi, and are useful as an active ingredient of agricultural or horticultural fungicides. The compounds of the present invention also have high controlling activities on larvae and imagoes of (1) *Lepidoptera*, such as *Spodoptera litura, Chilo suppressalis, Cuapha locrocis medinalis, Plutella xylostella*, and *Adoxophyes* sp., (2) *Hemiptera*, such as *Delphacidae* (e.g., *Sogatella furcifera*, *Nilaparvata lugens*, and *Laodelphax striatellus*), *Deltocephalidae* (e.g., *Nephotettix cincticeps*, *Empoasca onukii*, and *Tettigella viridis*), *Aphididae* (e.g., *Myzus persicae* and *Aphis gossypii*), *Aleyrodidae* (e.g., *Trialeurodes vaporariorum*), and *Pentahomidae* (e.g., *Plautia stali*), (3) *Coleoptera*, such as *Phyllotreta striolata*, *Aulacophora femoralis*, and *Callosobruchus chinensis*, (4) *Diptera*, such as *Musca domestica*, *Aedes aegypti*, and *Culex pipiens pallens*, and (5) *Orthoptera*, such as *Periplaneta americana*, and also on eggs, larvae and imagoes of *Acarina*, such as *Tetranychus urticae*, *Tetranychus cinnabarinus*, and *Panonychus citri*, and are useful as an active ingredient of agricultural and horticultural insecticides and acaricides. The plant pathogens and insects which are controllable by the compounds of the present invention are not limited to the above examples.

In using the compounds of the present invention as pesticides, they may be applied as such but are preferably formulated into pesticidal compositions together with appropriate adjuvants customarily employed in the art. While not limiting, the forms of the pesticides include emulsifiable concentrates, wettable powders, dusts, flowables, powders, granules, tablets, oils, sprays, and fumigants. These preparations can comprise one or more than one compounds of the present invention.

The adjuvants are used in the pesticides for the purpose of improvement of the pesticidal effect, stabilization of the

preparation, improvement of dispersibility, and the like. Useful adjuvants include carriers (diluents), spreaders, emulsifiers, wetting agents, dispersants, and disintegrants. Suitable liquid carriers include water; aromatic hydrocarbons such as toluene and xylene; alcohols such as methanol, butanol and glycol; ketones such as acetone; amides such as dimethylformamide; sulfoxides such as dimethyl sulfoxide; methylnaphthalene; cyclohexane; animal or vegetable oils; and fatty acids. Examples of suitable solid carriers are clay, kaolin, talc, diatomaceous earth, silica, calcium carbonate, montmorillonite, bentonite, feldspar, quartz, alumina, sawdust, nitrocellulose, starch, and gum arabic. General surface active agents serve as an emulsifier or a dispersant. For example, anionic, cationic, nonionic or amphoteric surface active agents, such as sodium higher alcohol sulfates, stearyltrimethylammonium chloride, polyoxyethylene alkyl phenyl ethers, and lauryl betaine, are useful. Further, spreaders, such as polyoxyethylene nonylphenyl ether and polyoxyethylene laurylphenyl ether; wetting agents such as dialkyl sulfosuccinates; fixing agents, such as carboxymethyl cellulose and polyvinyl alcohol; and disintegrants, such as sodium lignin sulfonate and sodium laurylsulfate can be used.

The concentration of the active ingredient in the pesticides is selected from the range of from 0.1 to 99.5% by weight, being appropriately decided in accordance with the form of the preparation, the method of application or other conditions. For example, a suitable active ingredient concentration is about 0.5 to 20% by weight, preferably 1 to 10% by weight, in dusts; about 1 to 90% by weight, preferably 10 to 80% by weight, in wettable powders; or about 1 to 90% by weight, preferably 10 to 40% by weight, in emulsifiable concentrates.

Emulsifiable concentrates are prepared by mixing the compound of the present invention as an active ingredient with a solvent, a surface active agent, etc. The emulsifiable concentrate can be applied as diluted with water to a prescribed concentration. Wettable powders are prepared by mixing the active ingredient with a solid carrier, a surface active agent, etc. The wettable powder can be applied as suspended in water in a prescribed concentration. Dusts are prepared by mixing the active ingredient with solid carriers and can be applied as such. Powders or granules are prepared by mixing the active ingredient with solid carriers, surface active agents, etc., followed by granulation. Powders or granules can be applied as such. The above description about the methods for preparing the pesticidal compositions is not intended to limit the present invention, and one skilled in the art can select an appropriate method in accordance with the active ingredient, the purpose of application, and the like.

The pesticides of the present invention comprising the compound of the present invention as an active ingredient can further contain arbitrarily other pesticidal compounds (inclusive of herbicides), insect growth regulators, fertilizers, soil conditioners, and so on.

The usage of the pesticides according to the present invention is not particularly restricted. That is, the pesticide can be used for foliar spray treatment, submerged application, pre-emergence soil treatment, seed treatment, and the like. For foliar spray treatment, a solution having a concentration of 5 to 1000 ppm, preferably 10 to 500 ppm, is applied in an amount of about 100 to 200 ℓ per 10 are. For submerged application, granules having an active ingredient content of 5 to 15% is scattered in an amount of 1 to 10 kg per 10 are. For soil treatment, a solution having a concentration of 5 to 1000 ppm is applied in an amount of about 1 to 10 ℓ per $m^2$. For seed treatment, a solution having a concentration of 10 to 1000 ppm is applied in an amount of about 10 to 100 ml per kg of seeds.

Best Modes for Carrying out the Invention:

The present invention will now be illustrated in greater detail with reference to Examples, but it should be understood that the present invention is not limited thereto.

REFERENCE EXAMPLE 1

In a 5 ℓ flask were put 2.4 ℓ of toluene and 154.8 g of methylhydrazine, and 379.2 g of ethyl acetylpyruvate was dropwise added thereto over 20 minutes while maintaining the reaction temperature at -5° to 0°C by cooling on a dry ice bath (-20 to -15°C). After the addition, the bath temperature was gradually raised to keep the reaction temperature at 8 to 10°C, at which the reaction mixture was stirred for 30 minutes to complete the reaction. The reddish brown aqueous phase was separated, washed with 300 ml of a saturated sodium chloride aqueous solution, concentrated under reduced pressure, and distilled to give 309.5 g (yield: 61.4%) of ethyl 1,3-dimethylpyrazole-5-carboxylate having a boiling point of 81.5 to 83°C/4 mmHg.

REFERENCE EXAMPLE 2

In a 1 ℓ flask were charged 101.3 g of ethyl 1,3-dimethylpyrazole-5-carboxylate obtained in Reference Example 1 and 200 ml of methanol, and 39 g of chlorine, which had been liquefied on dry ice and weighed, was spontaneously vaporized and blown into the mixture while vigorously stirring. Blowing required 40 minutes. The reaction temperature reached 62°C. After the blowing, the reaction mixture was stirred for 30 minutes, followed by concentration under

reduced pressure to give 101.3 g of crude ethyl 1,3-dimethyl-4-chloropyrazole-5-carboxylate.

REFERENCE EXAMPLE 3

In a 1 ℓ flask, 20.8 g of 95% sodium hydroxide was dissolved in 320 ml of water. To the solution was added 91.9 g of ethyl 1,3-dimethyl-4-chloropyrazole-5-carboxylate prepared in Reference Example 2. The mixture was vigorously stirred on an oil bath at 90°C for 1 hour to conduct the reaction (reaction temperature: 83°C). After cooling to room temperature, the reaction mixture was neutralized with diluted hydrochloric acid prepared from 50 ml of concentrated hydrochloric acid and 200 ml of water. The precipitate thus formed was collected by filtration, washed with water, and concentrated under reduced pressure to give 72.2 g (yield: 92.0%) of crude 1,3-dimethyl-4-chloropyrazole-5-carboxylic acid.

REFERENCE EXAMPLE 4

In a 100 ml flask were charged 9.3 g of 1,3-dimethyl-4-chloropyrazole-5-carboxylic acid obtained in Reference Example 3, 30 ml of toluene, and 11.9 g of thionyl chloride, and the mixture was heated under reflux for 2 hours on an oil bath. Concentration of the reaction mixture gave 9.3 g (yield: 91%) of crude 1,3-dimethyl-4-chloropyrazole-5-carboxylic acid chloride.

REFERENCE EXAMPLE 5

To a solution of 2.9 g of β-naphthol in 15 ml of dimethylformamide was slowly added 0.8 g of 60% sodium hydride, followed by stirring at room temperature for 30 minutes. To the mixture was added slowly 2.77 g of 2-chloro-5-cyanopyridine, followed by stirring at 40°C for 1 hour. After cooling to room temperature, 50 ml of ethyl acetate and 50 ml of water were added thereto. The extracted organic layer was washed with a saturated sodium chloride aqueous solution and dried over anhydrous sodium sulfate. Any insoluble matter was separated by filtration, and the filtrate was concentrated. The residue was purified by column chromatography on silica gel (developing solvent: hexane/ethyl acetate=4/1) to give 4.14 g of 2-(β-naphthyloxy) 5-cyanopyridine.

REFERENCE EXAMPLE 6

To a solution of 1.72 g of 2-(β-naphthyloxy)-5-cyanopyridine obtained in Reference Example 5 in 50 ml of ethanol were added 1.7 g of 28% aqueous ammonia and 1.5 g of Raney nickel to perform hydrogenation. After completion of the reaction, Raney nickel was separated by filtration, and the solvent was evaporated from the filtrate to recover crude 5-aminomethyl-2-(β-naphthyloxy)pyridine.

EXAMPLE 1

Preparation of N-[6-(3-Trifluoromethylphenoxy)-3-pyridylmethyl]-1,3,4-trimethyl-5-pyrazolecarboxamide

A mixture of 7.7 g of 1,3,4-trimethyl-5-pyrazolecarboxylic acid and 12 g of thionyl chloride was heated under reflux for 1 hour. Thionyl chloride was removed by evaporation under reduced pressure, and the residue was dissolved in 20 ml of toluene. The resulting solution was added dropwise to a solution of 13.4 g of 5-aminomethyl-2-(3-trifluoromethylphenoxy)pyridine and 6.1 g of triethylamine in 30 ml of toluene at 0 to 10°C. After the addition, the reaction mixture was stirred at room temperature for 2 hours. The reaction mixture was poured into ice-water and extracted with toluene. The toluene layer was washed successively with a sodium hydrogencarbonate aqueous solution, water and a saturated sodium chloride aqueous solution and dried over anhydrous sodium sulfate. The solvent was removed by evaporation under reduced pressure, and the residue was purified by silica gel column chromatography to give 18.2 g of compound No. 10 shown in Table 1 below.

EXAMPLE 2

The compounds shown in Table 1 were obtained in the same manner as in Example 1. In Table 1, the values with an asterisk in the column "Melting Point" are refractive indices.

TABLE 1

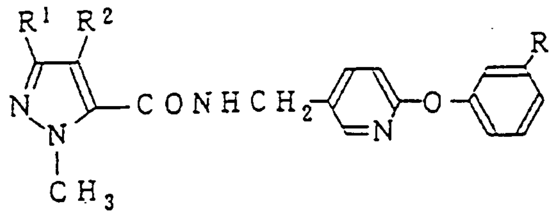

| Compound No. | $R^1$ | $R^2$ | $R^6$ | M. P. (°C) |
|---|---|---|---|---|
| 1 | H | H | $CF_3$ | 126-128 |
| 2 | H | Cl | $CF_3$ | 87-88 |
| 3 | $CH_3$ | H | $CF_3$ | 1.5424*(25℃) |
| 4 | $CH_3$ | Cl | $CF_3$ | 118-119 |
| 5 | $CH_3$ | Br | $CF_3$ | 119-120 |
| 6 | $CH_3$ | I | $CF_3$ | 135-136 |
| 7 | $CH_3$ | $CH_3$ | $CH_3$ | 116-117 |
| 8 | $CH_3$ | $CH_3$ | Cl | 126-127 |
| 9 | $CH_3$ | $CH_3$ | Br | 118-120 |
| 10 | $CH_3$ | $CH_3$ | $CF_3$ | 125-126 |
| 11 | $CH_3$ | $CH_3$ | $OCHF_2$ | 106-107 |
| 12 | $C_2H_5$ | H | $CF_3$ | 1.5518*(25℃) |
| 13 | $C_2H_5$ | Cl | $CF_3$ | 114-115 |
| 14 | $C_2H_5$ | Br | $CF_3$ | 127-128 |
| 15 | $C_2H_5$ | $CH_3$ | $CF_3$ | 121-122 |
| 16 | iso-$C_3H_7$ | Cl | $CF_3$ | 107-108 |
| 17 | $C_6H_5$ | H | $CF_3$ | 1.5758*(24℃) |
| 18 | 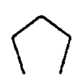 | | $CF_3$ | 119-120 |
| 19 | 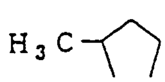 | | $CF_3$ | 133-135 |
| 20 | $CF_3$ | H | $CF_3$ | 97-98 |

The NMR and IR spectra of Compound Nos. 3, 10, 12, and 17 were as follows.

Compound No. 3

$^1$H-NMR($CDCl_3$) δ ppm: 2.24 (3H, s), 4.11 (3H, s), 4.54 (2H, d), 6.27 (1H, s), 6.37 (1H, bs), 6.96 (1H, d), 7.31 (1H, d), 7.39 (1H, s), 7.49 (2H, m), 7.75 (1H, dd), 8.12 (1H, d)
IR(KBr) $cm^{-1}$: 3320, 2940, 1660

Compound No. 10

$^1$H-NMR($CDCl_3$) δ ppm: 2.12 (3H, s), 2.18 (3H, s), 4.02 (3H, s), 4.60 (2H, d), 6.08 (1H, bs), 6.98 (1H, d), 7.31 (1H, d), 7.40 (1H, bs), 7.50 (2H, m), 7.78 (1H, dd), 8.16 (1H, d)
IR(KBr) $cm^{-1}$: 3270, 1630, 1480, 1320, 1120, 920, 830

Compound No. 12

$^1$H-NMR($CDCl_3$) δ ppm: 1.22 (3H, t), 2.62 (2H, q), 4.12 (3H, s), 4.55 (2H, d), 6.30 (1H, s), 6.33 (1H, bs), 6.97 (1H, d), 7.32 (1H, d), 7.40 (1H, s), 7.49 (2H, m), 7.75 (1H, dd), 8.13 (1H, d)
IR(KBr) $cm^{-1}$: 3320, 2980, 1650, 1550, 1480, 1330, 1120, 920

Compound No. 17

$^1$H-NMR($CDCl_3$) δ ppm: 4.24 (3H, s), 4.59 (2H, d), 6.38 (1H, bs), 6.77 (1H, s), 6.98 (1H, d), 7.4 (6H, m), 7.76 (3H, m), 8.18 (1H, s)
IR(KBr) $cm^{-1}$: 3330, 2950, 1660, 1550, 1480, 1320, 1120, 920

EXAMPLE 3

Preparation of N-[6-β-Naphthyloxy)-3-pyridyl-methyl]-4-chloro-1,3-dimethyl-5-pyrazolecarboxamide

In 10 ml of toluene was dissolved 0.56 g of 4-chloro-1,3-dimethyl-5-pyrazolecarboxylic acid chloride. The resulting solution was added dropwise to a solution of 0.73 g of 5-aminomethyl-2-(β-naphthyloxy)pyridine and 0.3 g of triethylamine in 10 ml of toluene at 0 to 10°C. After the addition, the reaction mixture was stirred at room temperature for 2 hours, and then poured into ice-water and extracted with toluene. The toluene layer was washed successively with a sodium hydrogencarbonate aqueous solution, water, and a saturated sodium chloride aqueous solution and dried over anhydrous sodium sulfate. The solvent was evaporated under reduced pressure, and the residue was purified by silica gel column chromatography to give 0.99 g of compound 22 (shown in Table 2).

EXAMPLE 4

The compounds shown in Tables 2 and 3 were obtained in accordance with the process of Example 3. In the Tables, the values with an asterisk are refractive indices.

TABLE 2

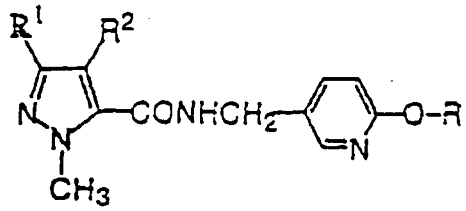

| Compound No. | $R^1$ | $R^2$ | R | M. Pt. (°C) or Refractive Index |
|---|---|---|---|---|
| 21 | $CH_3$ | Cl | 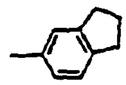 | 116-117 |
| 22 | $CH_3$ | Cl | 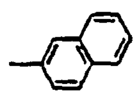 | 150-151 |
| 23 | $CH_3$ | Br | 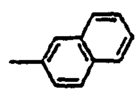 | 151-152 |
| 24 | $CH_3$ | $CH_3$ | 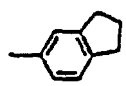 | 121-122 |
| 25 | $CH_3$ | $CH_3$ | 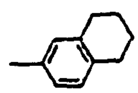 | 119-120 |
| 26 | $CH_3$ | $CH_3$ | 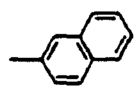 | 153-155 |
| 27 | $CH_3$ | $CH_3$ | 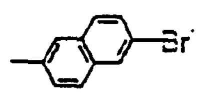 | 156-158 |

TABLE 2 (cont'd)

| Compound No. | $R^1$ | $R^2$ | R | M. Pt. (°C) or Refractive Index |
|---|---|---|---|---|
| 28 | $CH_3$ | $CH_3$ | 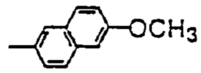 | 154–155 |
| 29 | $CH_3$ | $CH_3$ | 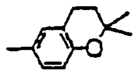 | 91–93 |
| 30 | $CH_3$ | $CH_3$ | 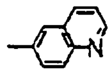 | 129–131 |
| 31 | $CH_3$ | $CH_3$ | 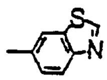 | 179–181 |
| 32 | $CH_3$ | OH | 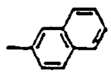 | amorphous solid |
| 33 | $CH_3$ | $OCH_3$ | 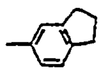 | 85–87 |
| 34 | $CH_3$ | $OCH_3$ | 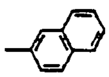 | 1.6109* (24 ℃) |
| 35 | $CH_3$ | $OCHF_2$ | 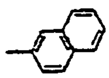 | 104–105 |
| 36 | $CH_3$ | 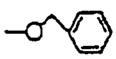 | 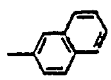 | 92–93 |
| 37 | $C_2H_5$ | Cl | 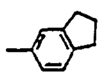 | 99–100 |
| 38 | $C_2H_5$ | Cl | 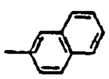 | 121–122 |
| 39 | $C_2H_5$ | Br | 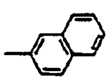 | 127–129 |
| 40 | $C_2H_5$ | $CH_3$ | 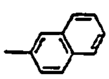 | 126–128 |
| 41 | $C_3H_7{}^{i}$ | Cl | 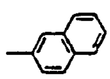 | 122–123 |
| 42 | 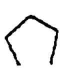 | | 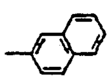 | 157–158 |
| 43 | 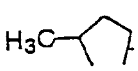 | | 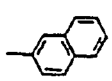 | 142–144 |

TABLE 3

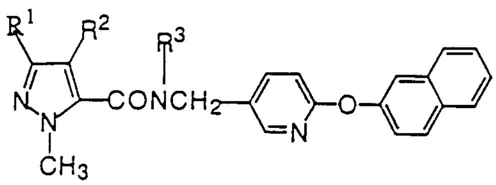

| Compound No. | $R^1$ | $R^2$ | $R^3$ | M. Pt. (°C) or Refractive Index |
|---|---|---|---|---|
| 44 | $CH_3$ | Cl | $CH_3$ | 1.6038*(25℃) |
| 45 | $C_2H_5$ | Cl | 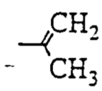 | 82-83 |
| 46 | $C_2H_5$ | Cl | 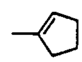 | viscous |
| 47 | $C_2H_5$ | Cl | 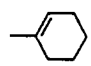 | viscous |
| 48 | $CH_3$ | $OCH_3$ | CHO | viscous |
| 49 | $C_2H_5$ | Cl | CHO | viscous |
| 50 | $CH_3$ | $CH_3$ | CHO | viscous |
| 51 | $CH_3$ | Cl | $COCH_3$ | 1.6010*(25℃) |
| 52 | $CH_3$ | $OCH_3$ | $COCH_3$ | 106 |
| 53 | $C_2H_5$ | Cl | $COCH_3$ | viscous |
| 54 | $CH_3$ | $CH_3$ | $COCH_3$ | viscous |
| 55 | $CH_3$ | $OCH_3$ | $COC_2H_5$ | viscous |
| 56 | $CH_3$ | $OCH_3$ | $COC_3H_7$ | viscous |
| 57 | $CH_3$ | $CH_3$ | $COC_3H_7$ | viscous |

TABLE 3 (cont'd)

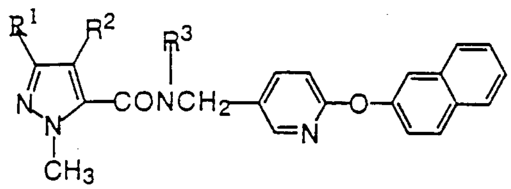

| Compound No. | $R^1$ | $R^2$ | $R^3$ | M. Pt. (°C) or Refractive Index |
|---|---|---|---|---|
| 58 | $CH_3$ | $OCH_3$ | $COC_6H_5$ | amorphous solid |
| 59 | $CH_3$ | $OCH_3$ | $COOCH_3$ | 104-106 |
| 60 | $C_2H_5$ | Cl | $COOCH_3$ | viscous |
| 61 | $CH_3$ | $OCH_3$ | $COOCH_2C_6H_5$ | amorphous solid |
| 62 | $CH_3$ | $OCH_3$ | $CH_2OCH_3$ | viscous |
| 63 | $CH_3$ | $OCH_3$ | $CH_2OC_2H_5$ | viscous |
| 64 | $CH_3$ | $OCH_3$ | $CH_2OCH_2C_6H_5$ | viscous |
| 65 | $CH_3$ | $OCH_3$ | $SO_2CH_3$ | viscous |
| 66 | $CH_3$ | $OCH_3$ | $SO_2C_6H_4$-p-$CH_3$ | viscous |
| 67 | $C_2H_5$ | Cl | $CH_3$ | 143-144 |
| 68 | $C_2H_5$ | Cl | $COC_2H_5$ | viscous |
| 69 | $C_2H_5$ | Cl | $COC_3H_7$ | viscous |
| 70 | $C_2H_5$ | Cl | $COC_6H_5$ | viscous |
| 71 | $C_2H_5$ | Cl | $SO_2C_6H_4$-p-$CH_3$ | viscous |

The NMR data of the compounds shown in Tables 2 and 3 were as follows.

Compound 21:

$^1$H-NMR($CDCl_3$) δ ppm: 2.11 (2H, m), 2.90 (4H, m), 4.13 (3H, s), 4.58 (2H, d), 6.89 (2H, d), 6.98 (1H, s), 7.00 (1H, bs), 7.22 (1H, d), 7.67 (1H, dd), 8.17 (1H, d)

Compound 22:

$^1$H-NMR($CDCl_3$) δ ppm: 2.23 (3H, s), 4.12 (3H, s), 4.59 (2H, d), 6.96 (1H, d), 7.00-7.10 (1H, bt), 7.29 (1H, dd), 7.40-7.50 (2H, m), 7.56 (1H, d), 7.71-7.76 (2H, m), 7.86 (2H, t), 8.18 (1H, d)

Compound 23:

$^1$H-NMR($CDCl_3$) δ ppm: 2.24 (3H, s), 4.13 (3H, s), 4.60 (2H, d), 6.97 (1H, d), 7.00-7.10 (1H, bt), 7.29 (1H, dd), 7.42-7.53 (2H, m), 7.56 (1H, d), 7.76 (2H, t), 7.86 (2H, t), 8.20 (1H, d)

Compound 24:

$^1$H-NMR($CDCl_3$) δ ppm: 2.10 (2H, m), 2.10 (3H, s), 2.17 (3H, s), 2.90 (4H, m), 4.01 (3H, s), 4.57 (2H, d), 6.00 (1H, bs), 6.90 (2H, dd), 6.98 (1H, s), 7.22 (1H, d), 7.70 (1H, dd), 8.15 (1H, d)

Compound 25:

$^1$H-NMR($CDCl_3$) δ ppm: 1.79 (4H, m), 2.10 (3H, s), 2.17 (3H, s), 2.76 (4H, bm), 4.01 (3H, s), 4.57 (2H, d), 6.00 (1H, bs), 6.85 (3H, m), 7.07 (1H, d), 7.68 (1H, dd), 8.17 (1H, s)

Compound 26:

$^1$H-NMR($CDCl_3$) δ ppm: 2.11 (3H, s), 2.17 (3H, s), 4.01 (3H, s), 4.59 (2H, d), 6.05 (1H, bs), 6.97 (1H, d), 7.28 (1H, dd), 7.46 (2H, m), 7.56 (1H, d), 7.70-7.90 (4H, m), 8.17 (1H, d)

Compound 27:

$^1$H-NMR($CDCl_3$) δ ppm: 2.12 (3H, s), 2.18 (3H, s), 4.02 (3H, s), 4.60 (2H, d), 6.05 (1H, bs), 6.99 (1H, d), 7.31 (1H, dd), 7.55 (2H, m), 7.64 (1H, d), 7.77 (2H, m), 8.01 (1H, s), 8.17 (1H, d)

Compound 28:

$^1$H-NMR($CDCl_3$) δ ppm: 2.11(3H, s), 2.18(3H, s), 3.92 (3H, s), 4.01 (3H, s), 4.59 (2H, d), 6.02 (1H, bs), 6.95 (1H, d), 7.16 (2H, m), 7.3 (1H, d), 7.50 (1H, d), 7.70 (2H, m), 7.78 (1H, d), 8.17 (1H, d)

Compound 29:

$^1$H-NMR($CDCl_3$) δ ppm: 1.34 (6H, s), 1.80 (2H, t), 2.10 (3H, s), 2.17 (3H, s), 2.77 (2H, t), 4.01 (3H, s), 4.57 (2H, d), 6.00 (1H, bs), 6.80 (3H, m), 7.68 (1H, dd), 8.17 (1H, d)

Compound 30:

$^1$H-NMR($CDCl_3$) δ ppm: 2.12 (3H, s), 2.18 (3H, s), 4.02 (3H, s), 4.60 (2H, d), 6.10 (1H, b), 7.03 (1H, d), 7.40 (1H, m), 7.52 (2H, m), 7.78 (1H, dd), 8.13 (3H, m), 8.88 (1H, t)

Compound 31:

$^1$H-NMR($CDCl_3$) δ ppm: 2.12 (3H, s), 2.18 (3H, s), 4.02 (3H, s), 4.60 (2H, d), 6.07 (1H, b), 6.99 (1H, d), 7.30 (1H, d), 7.74 (1H, dd), 7.78 (1H, dd), 8.15 (2H, d), 8.96 (1H, s)

Compound 32:

$^1$H-NMR($CDCl_3$) δ ppm: 2.09 (3H, s), 4.03 (3H, s), 4.52 (2H, d), 6.88 (1H, d), 7.22 (1H, dd), 7.45 (2H, m), 7.49 (1H,

d), 7.64 (1H, m), 7.72 (2H, m), 7.83 (2H, m), 8.10 (1H, s)

Compound 33:

$^1$H-NMR($CDCl_3$) δ ppm: 2.10 (2H, m), 2.28 (3H, s), 2.90 (4H, q), 3.81 (3H, s), 4.09 (3H, s), 4.54 (2H, d), 6.87 (2H, d), 6.98 (1H, bs), 7.22 (1H, d), 7.49 (1H, bs), 7.68 (1H, dd), 8.15 (1H, d)

Compound 34:

$^1$H-NMR($CDCl_3$) δ ppm: 2.28 (3H, s), 3.82 (3H, s), 4.10 (3H, s), 4.57 (2H, d), 6.96 (1H, d), 7.28 (1H, dd), 7.46 (2H, m), 7.53 (1H, bs), 7.58 (1H, d), 7.70-7.90 (4H, m), 8.17 (1H, d)

Compound 35:

$^1$H-NMR($CDCl_3$) δ ppm: 2.23 (3H, s), 4.11 (3H, s), 4.56 (2H, d), 6.35 (1H, t), 6.88 (1H, bs), 6.97 (1H, d), 7.28 (1H, dd), 7.46 (2H, m), 7.56 (1H, d), 7.70 (1H, d), 7.77 (1H, d), 7.86 (2H, t), 8.17 (1H, s)

Compound 36:

$^1$H-NMR($CDCl_3$) δ ppm: 2.27 (3H, s), 4.10 (3H, s), 4.37 (2H, d), 4.93 (2H, s), 6.87 (1H, d), 7.21 (2H, m), 7.34 (3H, m), 7.46 (3H, m), 7.56 (2H, m), 7.78 (1H, d), 7.86 (2H, m), 8.04 (1H, s)

Compound 37:

$^1$H-NMR($CDCl_3$) δ ppm: 1.23 (3H, t), 2.11 (2H, m), 2.63 (2H, q), 2.90 (4H, m), 4.13 (3H, s), 4.58 (2H, q), 6.89 (2H, d), 6.98 (1H, d), 7.00 (1H, bs), 7.22 (1H, d), 7.69 (1H, dd), 8.18 (1H, d)

Compound 38:

$^1$H-NMR($CDCl_3$) δ ppm: 1.23 (3H, t), 2.63 (2H, q), 4.14 (3H, s), 4.60 (2H, d), 6.97 (1H, d), 7.00-7.20 (1H, bt), 7.27 (1H, dd), 7.40-7.50 (2H, m), 7.56 (1H, d), 7.76 (2H, m), 7.86 (2H, t), 8.19 (1H, d)

Compound 39:

$^1$H-NMR($CDCl_3$) δ ppm: 1.23 (3H, t), 2.62 (2H, q), 4.14 (3H, s), 4.61 (2H, d), 6.97 (1H, d), 7.05 (1H, bs), 7.28 (1H, dd), 7.46 (2H, m), 7.55 (1H, d), 7.76 (2H, m), 7.86 (2H, t), 8.20 (1H, s)

Compound 40:

$^1$H-NMR($CDCl_3$) δ ppm: 1.20 (3H, t), 2.13 (3H, s), 2.57 (2H, q), 4.02 (3H, s), 4.60 (2H, d), 6.06 (1H, bs), 6.97 (1H, d), 7.29 (1H, d), 7.46 (2H, m), 7.56 (1H, d), 7.77 (2H, m), 7.86 (2H, t), 8.18 (1H, d)

Compound 41:

$^1$H-NMR($CDCl_3$) δ ppm: 1.28 (6H, d), 3.03 (1H, m), 4.13 (3H, s), 4.60 (2H, d), 6.97 (1H, d), 7.05 (1H, bs), 7.31 (1H, m), 7.46 (2H, m), 7.56 (1H, d), 7.76 (2H, m), 7.86 (2H, t), 8.19 (1H, d)

Compound 42:

$^1$H-NMR($CDCl_3$) δ ppm: 2.46 (2H, m), 2.72 (4H, m), 4.15 (3H, s), 4.56 (2H, d), 6.02 (1H, bs), 6.96 (1H, d), 7.27 (1H, dd), 7.46 (2H, m), 7.55 (1H, d), 7.75 (2H, m), 7.86 (2H, t), 8.15 (1H, d)

Compound 43:

$^1$H-NMR($CDCl_3$) δ ppm: 1.27 (3H, d), 2.00 (1H, m), 2.68 (3H, m), 3.12 (1H, m), 4.16 (3H, s), 4.56 (2H, d), 6.00 (1H, bs), 6.96 (1H, d), 7.28 (1H, dd), 7.46 (2H, m), 7.56 (1H, d), 7.76 (2H, m), 7.86 (2H, t), 8.15 (1H, d)

Compound 44:

$^1$H-NMR($CDCl_3$) δ ppm: 2.22 (3H, s), 2.98+3.03 (3H, s+s), 3.83+3.80 (3H, s+s), 4.71+4.52 (2H, s+b), 6.97 (1H, t), 7.30 (1H, dd), 7.47 (2H, m), 7.58 (1H, m), 7.78 (2H, m), 7.90 (2H, m), 8.18 (1H, d)

Compound 45:

$^1$H-NMR($CDCl_3$) δ ppm: 1.24 (3H, t), 2.63 (2H, q), 3.92 (3H, s), 4.99 (2H, s), 6.92 (1H, d), 7.28 (1H, dd), 7.45 (2H, m), 7.55 (1H, d), 7.75-7.83 (3H, m), 7.87 (1H, d), 7.29 (1H, d), 8.89 (1H, s)

Compound 46:

$^1$H-NMR($CDCl_3$) δ ppm: 1.23 (3H, s), 1.83 (2H, m), 2.19 (2H, bm), 2.30 (2H, bm), 3.81 (3H, s), 4.82 (2H, s), 5.14 (1H, bs), 6.95 (1H, d), 7.29 (1H, dd), 7.45 (2H, m), 7.57 (1H, d), 7.77 (2H, t), 7.86 (2H, t), 8.13 (1H, d)

Compound 47:

$^1$H-NMR($CDCl_3$) δ ppm: 1.26 (3H, s), 1.42 (2H, bm), 1.88 (2H, b), 1.99 (2H, b), 2.59 (2H, q), 3.78 (3H, s), 4.78 (2H, s), 5.29 (1H, bs), 6.95 (1H, d), 7.30 (1H, dd), 7.46 (2H, m), 7.57 (1H, d), 7.80 (2H, t), 7.86 (2H, t), 8.14 (1H, s)

Compound 48:

$^1$H-NMR($CDCl_3$) δ ppm: 2.25 (3H, s), 3.59 (3H, s), 3.91 (3H, s), 4.97 (2H, s), 6.90 (1H, d), 7.25 (1H, dd), 7.44 (2H, m), 7.53 (1H, d), 7.7-7.9 (4H, m), 8.30 (1H, d), 8.87 (1H, s)

Compound 49:

$^1$H-NMR($CDCl_3$) δ ppm: 1.24 (3H, t), 2.63 (2H, q), 3.92 (3H, s), 4.99 (2H, s), 6.92 (1H, d), 7.28 (1H, dd), 7.45 (2H, m), 7.55 (1H, d), 7.75-7.83 (3H, m), 7.87 (1H, d), 7.29 (1H, d), 8.89 (1H, s)

Compound 50:

$^1$H-NMR($CDCl_3$) δ ppm: 1.87 (3H, t), 2.18 (3H, s), 3.85 (3H, s), 4.98 (2H, s), 6.92 (1H, d), 7.27 (1H, dd), 7.46 (2H, m), 7.56 (1H, d), 7.76-7.89 (4H, m), 8.31 (1H, d), 8.86 (1H, s)

Compound 51:

$^1$H-NMR($CDCl_3$) δ ppm: 2.24 (3H, s), 2.28 (3H, s), 3.79 (3H, s), 4.95 (2H, b), 6.92 (1H, d), 7.26 (1H, m), 7.46 (2H, m), 7.55 (1H, d), 7.65 (1H, dd), 7.78 (1H, d), 7.86 (2H, m), 8.02 (1H, d)

Compound 52:

$^1$H-NMR($CDCl_3$) δ ppm: 2.23 (3H, s), 2.25 (3H, s), 3.66 (3H, s), 3.84 (3H, s), 4.98 (2H, s), 6.89 (1H, d), 7.24 (1H, d), 7.45 (2H, m), 7.53 (1H, d), 7.70 (1H, dd), 7.77 (1H, d), 7.84 (2H, m), 8.09 (1H, d)

Compound 53:

$^1$H-NMR($CDCl_3$) δ ppm: 1.24 (3H, t), 2.58 (3H, s), 2.62 (2H, q), 3.78 (3H, s), 4.80-5.10 (2H, b), 6.90 (1H, d), 7.25 (1H, dd), 7.44 (2H, m), 7.54 (1H, d), 7.64 (1H, dd), 7.76 (1H, dd), 7.82 (1H, dd), 7.85 (1H, d), 8.01 (1H, d)

Compound 54:

$^1$H-NMR($CDCl_3$) δ ppm: 1.94 (3H, t), 2.16 (3H, s), 2.18 (3H, s), 3.77 (3H, s), 4.8-5.1 (2H, b), 6.90 (1H, d), 7.26 (1H, dd), 7.46 (2H, m), 7.54 (1H, d), 7.67 (1H, d), 7.77 (1H, d), 7.84 (1H, dd), 7.87 (1H, d), 8.05 (1H, d)

Compound 55:

$^1$H-NMR($CDCl_3$) δ ppm: 1.10 (3H, t), 2.44 (3H, s), 2.50 (2H, q), 3.65 (3H, s), 3.84 (3H, s), 4.97 (2H, s), 6.89 (1H,

d), 7.24 (1H, dd), 7.46 (2H, m), 7.52 (1H, d), 7.68-7.88 (4H, m), 8.09 (1H, d)

Compound 56:

$^1$H-NMR($CDCl_3$) δ ppm: 0.84 (3H, s), 1.62 (2H, m), 2.42 (3H, s), 2.47 (2H, t), 3.66 (3H, s), 3.83 (3H, s), 4.96 (2H, s), 6.89 (1H, dd), 7.24 (1H, dd), 7.45 (2H, m), 7.52 (1H, d), 7.70 (1H, dd), 7.76 (1H, dd), 7.83 (1H, dd), 7.85 (1H, d), 8.09 (1H, d)

Compound 57:

$^1$H-NMR($CDCl_3$) δ ppm: 1.24 (3H, t), 2.63 (2H, q), 3.92 (3H, s), 4.99 (2H, s), 6.92 (1H, d), 7.28 (1H, dd), 7.45 (2H, m), 7.55 (1H, d), 7.75-7.83 (3H, m), 7.87 (1H, d), 7.29 (1H, d), 8.89 (1H, s)

Compound 58:

$^1$H-NMR($CDCl_3$) δ ppm: 1.93 (3H, s), 3.53 (3H, s), 3.75 (3H, s), 5.15 (2H, s), 6.93 (1H, d), 7.19-7.30 (4H, m), 7.39-7.50 (4H, m), 7.54 (1H, d), 7.74-7.88 (3H, m), 7.98 (1H, dd), 8.23 (1H, d)

Compound 59:

$^1$H-NMR($CDCl_3$) δ ppm: 2.05 (3H, s), 3.59 (3H, s), 3.68 (3H, s), 3.93 (3H, s), 4.93 (2H, s), 6.92 (1H, d), 7.27 (1H, dd), 7.46 (2H, m), 7.55 (1H, d), 7.74-7.90 (3H, m), 8.30 (1H, d)

Compound 60:

$^1$H-NMR($CDCl_3$) δ ppm: 1.22 (3H, t), 2.60 (2H, q), 3.69 (3H, s), 3.91 (3H, s), 4.94 (2H, bs), 6.93 (1H, d), 7.28 (1H, dd), 7.43 (2H, m), 7.56 (1H, d), 7.76 (1H, dd), 7.80-7.90 (3H, m), 8.28 (1H, d)

Compound 61:

$^1$H-NMR($CDCl_3$) δ ppm: 2.15 (3H, s), 3.54 (3H, s), 3.88 (2H, s), 4.94 (2H, s), 5.09 (2H, s), 6.89 (1H, d), 7.06 (2H, m), 7.24-7.30 (4H, m), 7.46 (2H, m), 7.54 (1H, d), 7.74-7.85 (4H, m), 8.29 (1H, d)

Compound 62:

$^1$H-NMR($CDCl_3$) δ ppm: 2.22 (3H, s), 3.01 (3H, s), 3.62 (3H, s), 3.81 (2H, s), 3.82 (2H, s), 4.72 (2H, s), 6.94 (1H, d), 7.29 (1H, dd), 7.46 (2H, m), 7.56 (1H, br), 7.77-7.89 (4H, m), 8.23 (1H, d)

Compound 63:

$^1$H-NMR($CDCl_3$) δ ppm: 1.09 (3H, t), 2.22 (3H, s), 3.23 (2H, q), 3.63 (3H, s), 3.81 (2H, s), 4.74 (2H, s), 4.77 (2H, s), 6.94 (1H, d), 7.29 (1H, dd), 7.48 (2H, m), 7.56 (1H, d), 7.77-7.89 (4H, m), 8.23 (1H, d)

Compound 64:

$^1$H-NMR($CDCl_3$) δ ppm: 2.18 (3H, s), 3.59 (3H, s), 3.83 (3H, s), 4.26 (2H, s), 4.77 (2H, s), 4.82 (2H, s), 6.91 (1H, d), 7.11 (1H, dd), 7.26-7.58 (7H, m), 7.72-7.88 (5H, m), 8.20 (1H, d)

Compound 65:

$^1$H-NMR($CDCl_3$) δ ppm: 2.25 (3H, s), 3.20 (3H, s), 3.76 (3H, s), 3.84 (3H, s), 5.07 (2H, s), 6.93 (1H, d), 7.24 (1H, dd), 7.44-7.55 (4H, m), 7.76-7.89 (3H, m), 7.98 (1H, d)

Compound 66:

$^1$H-NMR($CDCl_3$) δ ppm: 2.20 (3H, s), 2.43 (3H, s), 3.55 (3H, s), 3.74 (3H, s), 5.08 (2H, s), 6.85 (1H, d), 7.23 (1H, dd), 7.30 (2H, dd), 7.41-7.52 (4H, m), 7.55-7.88 (6H, m)

Compound 67:

$^1$H-NMR($CDCl_3$) δ ppm: 1.24 (3H, s), 2.62 (3H, s), 2.98 (3H, s), 3.84 (3H, s), 4.71 (2H, bs), 6.99 (1H, d), 7.30 (1H, dd), 7.38-7.52 (4H, m), 7.58 (1H, d), 7.87 (2H, t), 8.17 (1H, d)

Compound 68:

$^1$H-NMR($CDCl_3$) δ ppm: 1.16 (3H, t), 1.24 (3H, t), 2.54 (2H, q), 2.62 (2H, q), 3.80 (3H, s), 4.9-5.1 (2H, b), 6.91 (1H, d), 7.26 (1H, dd), 7.45 (2H, m), 7.54 (1H, d), 7.77 (1H, d), 7.84 (1H, d), 7.87 (1H, d), 8.01 (1H, d)

Compound 69:

$^1$H-NMR($CDCl_3$) δ ppm: 0.88 (3H, t), 1.24 (3H, t), 1.65 (2H, m), 2.49 (2H, t), 2.63 (2H, q), 3.79 (3H, s), 4.9-5.1 (2H, b), 6.91 (1H, d), 7.26 (1H, dd), 7.45 (2H, m), 7.54 (1H, d), 7.77 (1H, d), 7.84 (1H, d), 7.87 (1H, d), 8.01 (1H, d)

Compound 70:

$^1$H-NMR($CDCl_3$) δ ppm: 0.97 (3H, t), 2.33 (2H, q), 3.74 (3H, s), 5.17 (2H, bs), 6.94 (1H, d), 7.21-7.38 (4H, m), 7.4-7.5 (4H, m), 7.56 (1H, d), 7.77 (1H, dd), 7.84 (1H, d), 7.86 (1H, d), 7.96 (1H, dd), 8.39 (1H, d)

Compound 71:

$^1$H-NMR($CDCl_3$) δ ppm: 1.21 (3H, t), 2.44 (2H, q), 2.58 (2H, q), 3.59 (3H, s), 5.05 (2H, bs), 6.92 (1H, d), 7.25-7.34 (3H, m), 7.47 (2H, m), 7.4-7.5 (2H, m), 7.68 (2H, d), 7.79 (1H, d), 7.85 (1H, d), 7.88 (1H, d), 7.96 (1H, d), 7.98 (1H, dd), 8.39 (1H, d)

Formulation Examples of the agricultural and horticultural fungicides, bacteriocides, insecticides, and acaricides containing the compound of the present invention as an active ingredient are shown below for illustrative purposes only but not for limitation.

<u>FORMULATION EXAMPLE 1</u>

Wettable Powder:

Twenty parts of the compound of the present invention, 20 parts of Carplex #80 (white carbon produced by Shionogi & Co., Ltd.), 52 parts of ST Kaolin Clay (kaolinite produced by Tsuchiya Kaolin K.K.), 5 parts of Solpol 9047K (an anionic surface active agent produced by Toho Chemical Industry Co., Ltd.), and 3 parts of Runox P65L (an anionic surface active agent produced by Toho Chemical Industry Co., Ltd.) were mixed and ground uniformly to obtain a wettable powder containing 20% of the active ingredient.

<u>FORMULATION EXAMPLE 2</u>

Dust:

Two parts of the compound of the present invention, 93 parts of clay (produced by Nippon Talc K.K.), and 5 parts of Carplex #80 (white carbon produced by Shionogi & Co., Ltd.) were uniformly mixed and ground to obtain a dust containing 2% of the active ingredient.

<u>FORMULATION EXAMPLE 3</u>

Emulsifiable Concentrate:

In a mixed solvent of 35 parts of xylene and 30 parts of dimethylformamide was dissolved 20 parts of the compound of the present invention, and 15 parts of Solpol 3005X (a mixture of a nonionic surface active agent and an anionic surface active agent, produced by Toho Chemical Co., Ltd.) was added thereto to prepare an emulsifiable concentrate containing 20% of the active ingredient.

FORMULATION EXAMPLE 4

Flowable:

A mixture of 30 parts of the compound of the present invention, 5 parts of Solpol 9047K, 3 parts of Sorbon T-20 (a nonionic surface active agent produced by Toho Chemical Co., Ltd.), 8 parts of ethylene glycol, and 44 parts of water were wet ground in Dynomill (produced by shinmaru enterprises Co.). To the resulting slurry was added 10 parts of a 1% aqueous solution of xanthan gum (naturally occurring polymer), followed by mixing and grinding thoroughly to obtain a flowable containing 20% of the active ingredient.

TEST EXAMPLE 1

Insecticidal Effect on Larvae of *Nilaparvata lugens*:

A rice seedling was setted in a glass cylinder (inner diameter: 3 cm; length: 17 cm), and five 4th instar larvae of *Nilaparvata lugens* were set free therein. The agricultural and horticultural pesticide of the present invention (an emulsifiable concentrate) was prepared in accordance with Formulation Example 3 and diluted with water, and 0.5 ml of the resulting emulsion was sprayed in the cylinder by means of a spray tower (manufactured by Mizuho Rika) (duplicates at 1 concentration). Five days after the treatment, the mortality and agony of the larvae were examined to obtain a death rate (%) taking an agonizing insect as a 1/2 dead insect. The results obtained are shown in Table 4 (the compound numbers in Table 4 correspond to those in Tables 1, 2 and 3).

TABLE 4

| Compound No. | Concentration (ppm) | Death Rate (%) |
|---|---|---|
| 1 | 500 | 100 |
| 2 | 500 | 100 |
| 7 | 500 | 100 |
| 8 | 500 | 100 |
| 9 | 500 | 100 |
| 10 | 500 | 100 |
| 11 | 500 | 100 |
| 12 | 500 | 100 |
| 13 | 500 | 100 |
| 18 | 500 | 100 |
| 25 | 500 | 90 |
| 26 | 500 | 90 |
| 27 | 500 | 100 |
| 30 | 500 | 100 |
| 31 | 500 | 90 |
| 34 | 500 | 100 |
| 35 | 500 | 100 |

TABLE 4 (cont'd.)

| Compound No. | Concentration (ppm) | Death Rate (%) |
|---|---|---|
| 38 | 500 | 90 |
| 40 | 500 | 100 |
| 42 | 500 | 90 |
| 43 | 500 | 90 |
| 50 | 500 | 100 |
| 51 | 500 | 100 |
| 52 | 500 | 90 |
| 53 | 500 | 90 |
| 54 | 500 | 100 |
| 55 | 500 | 100 |
| 56 | 500 | 100 |
| 57 | 500 | 100 |
| 63 | 500 | 100 |

TEST EXAMPLE 2

Insecticidal Effect on Larvae of *Plutella xylostella*:

The agricultural and horticultural pesticide of the present invention (a wettable powder) was obtained in accordance with Formulation Example 1 and diluted with water to prepare a suspension having a prescribed concentration. A disc (6 cm in diameter) cut out of a cabbage leaf was soaked in the suspension for 1 minute, air-dried, and placed in a plastic cup (inner diameter: 7 cm). Five 3-instar larvae of *Plutella xylostella* were put in the cup (duplicates at one concentration). Four days later, the death and agony of the larvae were examined to obtain a death rate (%) taking an agonizing insect as a 1/2 dead insect. The results obtained are shown in Table 5 (the compound numbers in Table 5 correspond to those in Tables 1, 2 and 3).

TABLE 5

| Compound No. | Concentration (ppm) | Death Rate (%) |
|---|---|---|
| 4 | 500 | 100 |
| 5 | 500 | 100 |
| 7 | 500 | 100 |
| 10 | 500 | 100 |
| 11 | 500 | 100 |
| 12 | 500 | 100 |
| 13 | 500 | 100 |
| 18 | 500 | 100 |
| 25 | 500 | 90 |
| 27 | 500 | 90 |
| 28 | 500 | 100 |
| 31 | 500 | 100 |
| 34 | 500 | 100 |
| 38 | 500 | 90 |
| 39 | 500 | 90 |
| 40 | 500 | 100 |
| 42 | 500 | 100 |
| 43 | 500 | 100 |
| 51 | 500 | 100 |
| 62 | 500 | 100 |

TEST EXAMPLE 3

Acaricidal Effect on Imagoes of *Tetranychus urticae*:

Ten female imagoes of *Tetranychus urticae* were set free on a disc (3 cm in diameter) cut out of a kidney bean leaf. The agricultural and horticultural pesticide of the present invention (a wettable powder) was prepared in accordance with Formulation Example 1 and diluted with water to a prescribed concentration, and 3.5 ml of the resulting suspension was sprayed on the leaf disc by means of a rotary spray tower (manufactured by Mizuho Rika) (duplicates at one concentration). Twenty-four hours later dead mites were counted to obtain an imago death rate (%). The results obtained are shown in Table 6.

TEST EXAMPLE 4

Acaricidal Effect on Eggs of *Tetranychus urticae*:

Five female imagoes of *Tetranychus urticae* were set free on a disc (3 cm in diameter) of a kidney bean leaf for 20 hours to let the mites lay eggs and then removed. The agricultural and horticultural pesticide of the present invention (a wettable powder) was prepared in accordance with Formulation Example 1 and diluted with water to a prescribed concentration, and 3.5 ml of the resulting suspension was sprayed on the leaf disc by means of a rotary spray tower (manufactured by Mizuho Rika) (duplicates at one concentration). After 8 days from the application, hatched eggs and unhatched eggs were counted to obtain an egg death rate (%). The results obtained are shown in Table 6.

TABLE 6

| Compound No. | Concentration (ppm) | Imago Death Rate (%) | Egg Death Rate (%) |
|---|---|---|---|
| 2 | 500 | 100 | 100 |
| 12 | 500 | 100 | 100 |

TEST EXAMPLE 5

Fungicidal Effect on Rice Blast Fungus (*Magnaporthe grisea*):

Ten/pot rice plants (variety: Akinishiki) were cultivated in a resin pot (diameter: 6 cm). The agricultural and horticultural pesticide of the present invention (a wettable powder) was prepared in accordance with Formulation Example 1 and diluted with water to a prescribed concentration. The foliage of the plants at 3 to 4 leaf stage was sprayed with 10 ml/pot of the diluted preparation. After air drying, the plants were spray-inoculated with a spore suspension ($5.0 \times 10^5$ spores/ml) of *Magnaporthe grisea* having been cultured on an oatmeal medium, placed in a moist chamber at 25°C for 24 hours, and then allowed to stand in a greenhouse (22 to 25°C) for 7 days. The number of lesions appearing on the leaves were counted to obtain an average per leaf, and a control value was calculated according to the following formula. The results are shown in Table 7.

Control value (%) = (number of lesions per leaf in untreated pot - number of lesions per leaf in treated pot)/(number of lesions per leaf in untreated pot) x 100

TABLE 7

| Compound No. | Concentration (ppm) | Control Value (%) |
|---|---|---|
| 3 | 250 | 100 |
| 9 | 250 | 91 |
| 10 | 250 | 89 |
| 11 | 250 | 85 |
| 12 | 250 | 100 |
| 13 | 250 | 87 |
| 24 | 250 | 100 |
| 27 | 250 | 90 |
| 34 | 250 | 90 |
| 35 | 250 | 96 |
| 38 | 250 | 100 |
| 39 | 250 | 100 |
| 40 | 250 | 100 |
| 45 | 250 | 90 |
| 46 | 250 | 96 |
| 47 | 250 | 92 |
| 48 | 250 | 100 |
| 49 | 250 | 100 |
| 50 | 250 | 97 |
| 54 | 250 | 100 |
| 55 | 250 | 100 |
| 56 | 250 | 97 |
| 62 | 250 | 100 |
| 63 | 250 | 98 |

<u>TEST EXAMPLE 6</u>

Fungicidal Effect on Tomato Late Blight Fungus (*Phytophthora infestans*):

Three/pot tomato plants (variety: Red Cherry) were cultivated in a resin pot (diameter: 6 cm). The agricultural and horticultural pesticide of the present invention (a wettable powder) was prepared in accordance with Formulation Example 1 and diluted with water to a prescribed concentration. The resulting suspension was sprayed to the foliage of the plants at 3 to 4 leaf stage in an amount of 10 ml per pot. After air drying the suspension, the plants were spray-inoculated with a suspension of zoosporandia (5.9 x $10^4$ zoosporandia/ml) of *Phytophthora infestans* having been cultured on tomato leaves cut off a tomato plant, and the inoculated plants were placed in a moist chamber at 25°C for 24 hours and then allowed to stand in a greenhouse (20 to 22°C) for 4 days. The total area of the lesions appearing on each leaf was measured and expressed in disease severity index. A degree of disease (%) was calculated from the disease severity indices, and a control value (%) was obtained therefrom. The results are shown in Table 8. Disease severity index:

0 No lesion.
1 Lesion area is 1/3 or less.
3 Lesion area is 1/3 to 2/3.
5 Lesion area is 2/3 or more.

$$\text{Degree of disease (\%)} = (0 \times n_0 + 1 \times n_1 + 3 \times n_1 + 5 \times n_1)/5(n_0 + n_1 + n_3 + n_5) \times 100$$

wherein $n_x$ is the number of leaves having a disease severity index of x.

$$\text{Control value (\%)} = \text{(degree of disease in untreated pot - degree of disease in treated pot)/degree of disease in untreated pot} \times 100$$

TABLE 8

| Compound No. | Concn. (ppm) | Control Value (%) |
|---|---|---|
| 3 | 250 | 100 |
| 4 | 250 | 94 |
| 5 | 250 | 100 |
| 6 | 250 | 91 |
| 7 | 250 | 95 |
| 8 | 250 | 100 |
| 9 | 250 | 100 |
| 10 | 250 | 100 |
| 11 | 250 | 92 |
| 12 | 250 | 89 |
| 13 | 250 | 85 |
| 18 | 250 | 100 |
| 22 | 250 | 100 |
| 24 | 250 | 100 |
| 26 | 250 | 100 |
| 27 | 250 | 100 |
| 28 | 250 | 100 |
| 32 | 250 | 100 |
| 34 | 250 | 100 |
| 35 | 250 | 93 |
| 39 | 250 | 100 |
| 40 | 250 | 100 |
| 41 | 250 | 100 |
| 42 | 250 | 100 |
| 43 | 250 | 100 |

TABLE 8 (cont'd)

| Compound No. | Concn. (ppm) | Control Value (%) |
|---|---|---|
| 46 | 250 | 97 |
| 48 | 250 | 100 |
| 52 | 250 | 100 |
| 54 | 250 | 96 |
| 55 | 250 | 92 |
| 56 | 250 | 90 |
| 62 | 250 | 100 |
| 64 | 250 | 96 |
| 66 | 250 | 99 |

TEST EXAMPLE 7

Acute Toxicity in Mice in Single Oral Administration:

Acute toxicity (p.o.) in mice of compound Nos. 8, 10, 13, 22, 23, 38, 42, and 52 was examined as follows. For comparison, compounds (IV), (V) and (VI) disclosed in JP-A-2-62876 were tested similarly.

Compound Nos. 8, 10, 22, 23, 38, and 42 (30 mg each) were each suspended in 10 ml of a 0.5% CMC-Na aqueous solution. Compound Nos. 13 and 52 and compounds (IV), (V) and (VI) (30 mg each) were each suspended in 10 ml of a 0.5% tragacanth gum aqueous solution. The suspension was orally given to a group each consisting of five 6-week-old CD-1 male mice (available from Charles River) at a dose of 10 ml-suspension per kg of the body weight. The number of dead mice was counted after 7 days from the administration to obtain a death rate. The results obtained are shown in Table 9 below.

TABLE 9

| Compound No. | Dose (mg-active ingredient/kg) | Death Rate (%) |
|---|---|---|
| 8 | 30 | 0 |
| 10 | 30 | 0 |
| 13 | 30 | 0 |
| 22 | 30 | 0 |
| 23 | 30 | 0 |
| 38 | 30 | 0 |
| 42 | 30 | 0 |
| 52 | 30 | 0 |
| (IV) | 30 | 100 |
| (V) | 30 | 100 |
| (VI) | 30 | 100 |

Industrial Applicability:

The pyrazole compounds of the present invention exhibit excellent controlling effects on various plant pathogenic fungi or bacteria, insect pests and mites and are of high safety. The pyrazole compounds of the present invention are very useful as an active ingredient of fungicides, bacteriocides, insecticides and acaricides.

## Claims

1. A pyrazole compound represented by formula (I):

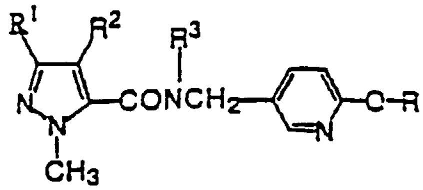

(I)

wherein $R^1$ represents a hydrogen atom, a $C_{1-4}$ alkyl group, a $C_{1-4}$ haloalkyl group, a $C_{1-4}$ alkoxy group or a phenyl group; $R^2$ represents a hydrogen atom, a halogen atom, a hydroxyl group, a $C_{1-4}$ alkyl group, a $C_{1-4}$ alkoxy group, a $C_{1-4}$ haloalkoxy group, a $C_{2-8}$ alkoxyalkoxy group or a benzyloxy group; or $R_1$ and $R_2$ are taken together to form a group:

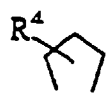

wherein $R^4$ represents a hydrogen atom or a $C_{1-4}$ alkyl group;
$R^3$ represents a hydrogen atom, a $C_{1-4}$ alkyl group, a $C_{2-5}$ alkenyl group, a $C_{5-7}$ cycloalkenyl group, a $C_{2-5}$ alkoxyalkyl group, a $C_{8-12}$ benzyloxyalkyl group, a formyl group, a $C_{2-5}$ alkanoyl group, a benzoyl group, a $C_{2-5}$ alkoxycarbonyl group, a benzyloxycarbonyl group, a $C_{1-4}$ alkylsulfonyl group or a $C_{6-10}$ arylsulfonyl group; and R represents a group represented by formulae:

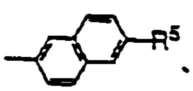, 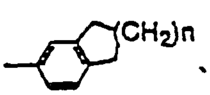, 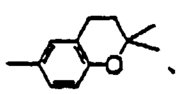, 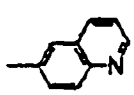, 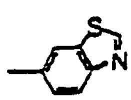 or 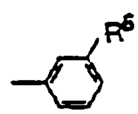

wherein $R^5$ represents a hydrogen atom, a halogen atom or a $C_{1-4}$ alkoxy group; $R^6$ represents a halogen atom, a $C_{1-4}$ alkyl group, a $C_{1-4}$ haloalkyl group, a $C_{1-4}$ alkoxy group or a $C_{1-4}$ haloalkoxy group; and n represents 1 or 2.

2. The pyrazole compound according to claim 1, wherein, in the general formula (I) shown in claim 1, R is a group represented by formula:

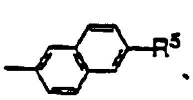, 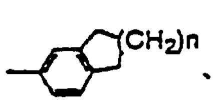, 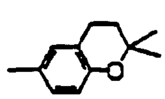, 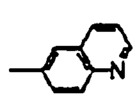 or 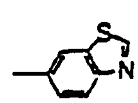

wherein $R^5$ represents a hydrogen atom, a halogen atom or a $C_{1-4}$ alkoxy group; and n represents 1 or 2.

3. The pyrazole compound according to claim 2, wherein, in the general formula (I) shown in claim 2, $R^1$ is a hydrogen atom or a $C_{1-4}$ alkyl group.

4. The pyrazole compound according to claim 2, wherein, in the general formula (I) shown in claim 2, $R^2$ is a halogen atom, a $C_{1-4}$ alkyl group or a $C_{1-4}$ alkoxy group.

5. The pyrazole compound according to claim 2, wherein, in the general formula (I) shown in claim 2, $R_1$ and $R_2$ are taken together to form a group represented by formula:

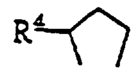

wherein $R^4$ represents a hydrogen atom or a $C_{1-4}$ alkyl group.

6. The pyrazole compound according to claim 2, wherein, in the general formula (I) shown in claim 2, $R^3$ is a hydrogen atom, a $C_{2-5}$ alkenyl group, a $C_{5-7}$ cycloalkenyl group, a $C_{2-5}$ alkoxyalkyl group, a benzyloxymethyl group, a formyl group, a $C_{2-5}$ alkanoyl group or a p-toluenesulfonyl group.

7. The pyrazole compound according to claim 2, wherein, in the general formula (I) shown in claim 2, $R^3$ is a hydrogen atom, a formyl group or an alkanoyl group.

8. The pyrazole compound according to claim 1, wherein, in the general formula (I) shown in claim 1, R is a group represented by formula:

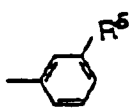

wherein $R^6$ is a halogen atom, a $C_{1-4}$ alkyl group, a $C_{1-4}$ haloalkyl group, a $C_{1-4}$ alkoxy group or a $C_{1-4}$ haloalkoxy group.

9. The pyrazole compound according to claim 8, wherein, in the general formula (I) shown in claim 8, $R^1$ is a $C_{1-4}$ alkyl group or a $C_{1-4}$ haloalkyl group.

10. The pyrazole compound according to claim 8, wherein, in the general formula (I) shown in claim 8, $R^2$ is a hydrogen atom, a halogen atom, a $C_{1-4}$ alkyl group or a $C_{1-4}$ alkoxy group.

11. The pyrazole compound according to claim 8, wherein, in the general formula (I) shown in claim 8, $R_1$ and $R_2$ are taken together to form a group represented by formula:

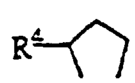

wherein $R^4$ represents a hydrogen atom or a $C_{1-4}$ alkyl group.

12. The pyrazole compound according to claim 8, wherein, in the general formula (I) shown in claim 8, $R^3$ is a hydrogen atom, a $C_{2-5}$ alkenyl group, a $C_{5-7}$ cycloalkenyl group, a $C_{2-5}$ alkoxyalkyl group, a benzyloxymethyl group, a formyl group, a $C_{2-5}$ alkanoyl group or a p-toluenesulfonyl group.

**13.** The pyrazole compound according to claim 8, wherein, in the general formula (I) shown in claim 2, $R^3$ is a hydrogen atom, a formyl group or an alkanoyl group.

**14.** The pyrazole compound according to claim 8, wherein, in the general formula (I) shown in claim 8, $R^6$ is a halogen atom, a $C_{1-4}$ alkyl group, a $C_{1-4}$ haloalkyl group, a $C_{1-4}$ alkoxy group or a $C_{1-4}$ haloalkoxy group.

**15.** The pyrazole compound according to claim 8, wherein, in the general formula (I) shown in claim 8, $R^6$ is a halogen atom or a $C_{1-4}$ haloalkyl group.

**16.** A pesticide comprising the pyrazole compound according to claim 1.

**17.** A fungicide or bacteriocide comprising the pyrazole compound according to claim 1.

**18.** An insecticide comprising the pyrazole compound according to claim 1.

**19.** An acaricide comprising the pyrazole compound according to claim 1.

## INTERNATIONAL SEARCH REPORT

International application No.
PCT/JP97/01162

**A. CLASSIFICATION OF SUBJECT MATTER**

Int. $Cl^6$ C07D401/12, 14, 405/14, 417/14, A01N43/56, 78

According to International Patent Classification (IPC) or to both national classification and IPC

**B. FIELDS SEARCHED**

Minimum documentation searched (classification system followed by classification symbols)

Int. $Cl^6$ C07D401/12, 14, 405/14, 417/14, A01N43/56, 78

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| X<br><br><br><br>A | JP, 2-62876, A (Mitsubishi Chemical Corp.), March 2, 1990 (02. 03. 90), Full descriptions & EP, 329090, A & US, 4968805, A | 1, 8-12, 14-19<br><br><br>2-7, 13 |
| P,A | JP, 8-277287, A (Mitsubishi Chemical Corp.), October 22, 1996 (22. 10. 96), Claim & EP, 726266, A & AU, 9643311, A | 1 - 19 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| June 16, 1997 (16. 06. 97) | June 24, 1997 (24. 06. 97) |
| Name and mailing address of the ISA/<br>Japanese Patent Office<br>Facsimile No. | Authorized officer<br><br>Telephone No. |

Form PCT/ISA/210 (second sheet) (July 1992)